# EUROPEAN PATENT SPECIFICATION

(11) **EP 3 220 877 B1**
(45) Date of publication and mention of the grant of the patent: **22.08.2018**
(21) Application number: 15787633.5
(22) Date of filing: 02.11.2015
(51) Int. Cl.: A61K 8/25, A61K 8/26, A61K 8/28, A61K 8/58, A61Q 15/00, A61K 8/898, A61K 8/92

(54) **ANTIPERSPIRANT COMPOSITION**
SCHWEISSHEMMENDE ZUSAMMENSETZUNG
COMPOSITION ANTI-TRANSPIRANTE

(30) Priority: 20.11.2014 WO PCT/CN2014/091795; 09.01.2015 EP 15150655
(43) Date of publication of application: 27.09.2017
(73) Proprietor: Unilever NV, 3013 AL Rotterdam (NL); Unilever PLC, London, Greater London EC4Y 0DY (GB)
(72) Inventor: YUAN, Su, Shanghai 200335 (CN); WATERFIELD, Philip, Christopher, Wirral Merseyside CH63 3JW (GB); ZHANG, Qiqing, Shanghai 200335 (CN)
(74) Representative: Whaley, Christopher
(86) International application number: PCT/EP2015/075419
(87) International publication number: WO 2016/078898

(56) References cited:
- WO-A1-2013/064367
- WO-A1-2014/147739
- JP-A- 2014 047 186
- US-A1- 2009 232 746
- DATABASE GNPD [Online] MINTEL; 1 April 2012 (2012-04-01), "Anti-Perspirant Deodorant Roll-on", XP002739559, Database accession no. 1769992
- DATABASE GNPD [Online] MINTEL; 1 November 2014 (2014-11-01), Anti-Perspirant Deodorant Roll-On: XP002739560, Database accession no. 2692765
- Anonymous: "Amodimethicone - Surfactants - SAA Pedia", , 11 October 2012 (2012-10-11), XP055189128, Retrieved from the Internet: URL:http://www.saapedia.org/en/saa/?type=d etail&id=1885 [retrieved on 2015-05-13]
- Anonymous: "Aluminum Zirconium Chlorohydrex Complexes with Glycine | Cosmetics Info", , 7 December 2015 (2015-12-07), XP055234010, Retrieved from the Internet: URL:http://www.cosmeticsinfo.org/ingredien t/aluminum-zirconium-chlorohydrex-complexe s-glycine [retrieved on 2015-12-07]
- "Teacher's Guide for (Under) Arm", , 1 April 2014 (2014-04-01), XP055234066, Retrieved from the Internet: URL:http://www.acs.org/content/dam/acsorg/ education/resources/highschool/chemmatters /teacherguide/chemmatters-tg-april2014-deo dorant.doc [retrieved on 2015-12-07]

## Description

### Technical Field of the Invention

The present invention is in the field of cosmetic compositions, in particular antiperspirant compositions and their use in reducing perspiration. Moreover, the present invention also relates to the process for manufacturing such antiperspirant compositions.

### Background of the Invention

Antiperspirant compositions are widely used by consumers to reduce perspiration by applying to the surface of the body, particularly to the underarm regions of the human body. However, antiperspirant compositions often comprise antiperspirant actives that are typically astringent metal salts such as aluminium or zirconium salts. Such salts can lead to irritation upon application to the surface of the human body by some consumers. Therefore, there is a need to provide an antiperspirant composition that is milder for use and also achieves good antiperspirant efficacy.

The present inventors have now developed an antiperspirant composition comprising hydrophobically modified particles, amino functionalized silicone, water and an antiperspirant active. It has been found that such composition can efficiently decrease the irritation while still achieving good sweat reduction. JP 2014 047186 A discloses anti-irritant antitranspirant compositions. Additionally, the composition can be formulated even when the composition is substantially free of emulsifiers, which further decreases the irritation.

### Summary of the Invention

In a first aspect, the present invention is concerned with an antiperspirant composition comprising:
a) hydrophobically modified particles selected from the group consisting of silica, metal oxide and mixtures thereof;
b) amino functionalized silicone;
c) water; and
d) an antiperspirant active
wherein the antiperspirant active is aluminum sesquichlorohydrate.

In a second aspect, the present invention is directed to a packaged antiperspirant product comprising the antiperspirant composition of the first aspect of this invention.

In a third aspect, the present invention is also directed to a process for manufacturing an antiperspirant composition of the first aspect, wherein the process comprises the steps of:
a) preparing an oil phase comprising hydrophobically modified particles selected from the group consisting of silica, metal oxide and mixtures thereof, and amino functionalized silicone;
b) preparing an aqueous phase comprising antiperspirant actives;
c) mixing the two phases to form the antiperspirant composition.

In a preferred embodiment of the third aspect, other optional ingredients are incorporated into the oil phase or aqueous phase during the preparation of either phase.

In another preferred embodiment of the third aspect, other optional ingredients are incorporated into the mixture of oil and aqueous phases to form the final antiperspirant composition.

In a fourth aspect, the present invention is concerned with an antiperspirant composition obtainable and/or obtained by a process of the third aspect.

In a fifth aspect, the present invention is directed to a method of using the antiperspirant composition of any embodiment of the first and the fourth aspects of this invention to reduce perspiration.

All other aspects of the present invention will more readily become apparent upon considering the detailed description and examples which follow.

Except in the examples, or where otherwise explicitly indicated, all numbers in this description indicating amounts of material or conditions of reaction, physical properties of materials and/or use may optionally be understood as modified by the word "about".

All amounts are by weight of the final antiperspirant composition, unless otherwise specified.

It should be noted that in specifying any ranges of values, any particular upper value can be associated with any particular lower value.

For the avoidance of doubt, the word "comprising" is intended to mean "including" but not necessarily "consisting of' or "composed of'. In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

### Detailed Description

It has been found that an antiperspirant composition comprising hydrophobically modified particles, amino functionalized silicone, water and an antiperspirant active is mild for use while still providing good antiperspirant efficacy.

Typically, the antiperspirant composition of the invention is a water-in-oil emulsion. Generally the water content is from 10 to 80%, more preferably from 15 to 70%, most preferably from 20 to 60% based on the total weight of the antiperspirant composition and including all ranges subsumed therein.

Preferably the composition is substantially free of emulsifiers. Substantially free of, as used herein, means less than 1.5%, and preferably less than 1.0%, and more preferably less than 0.75% and more preferably still less than 0.5%, and even more preferably less than 0.1% and most preferably from 0.0 to 0.01% by weight, based on total weight of the antiperspirant composition, including all ranges subsumed therein.

The antiperspirant compositions of the present invention are particularly suitable for dispensing from cosmetic roll-on dispensers that involve a container with a roll-on ball on a dispensing end.

### Hydrophobically Modified Particles

The only limitation with respect to the type of hydrophobically modified particles that may be used in this invention is that the same is suitable for use in an antiperspirant composition by consumers.

The hydrophobically modified particle includes silica, metal oxide or mixtures thereof. Preferably the hydrophobically modified particle is hydrophobically modified silica.

In a preferred embodiment, the hydrophobically modified silica comprises at least one of the following groups:

-O-Si(CH₃)₃ (I)

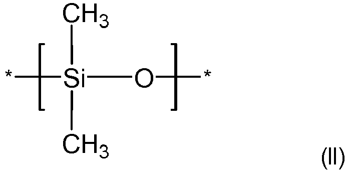

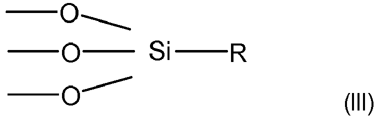

in which R is a C₄ to C₁₈ alkyl group.
or

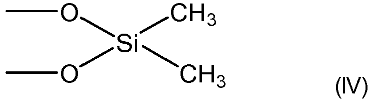

Such silicas are described in US 7 282 236 and made commercially available from suppliers like Evonik Degussa GmbH under the names Aerosil R805, R972, R202, R812, R104, R816 and R711. Preferably, the silica particles comprise the group representing by formula III. More preferably, the silica particles comprise octylsilane (sold under the name Aerosil R805).

The hydrophobically modified particles according to the present invention can be of different sizes and shapes. Particle size, as used herein, refers to particle diameter unless otherwise stated. Diameter is meant to mean the largest measurable distance on a particle in the event a well-defined sphere is not generated. Particle size can be measured, for example, by dynamic light scattering (DLS) or transmission electron microscope (TEM). The volume average primary particle size of hydrophobically modified particles is often from 1 nm to 100 nm, more preferably from 3 nm to 70 nm, most preferably from 5 nm to 20 nm, including all ranges subsumed therein.

Typically, the antiperspirant composition of the present invention comprises from 0.05 to 20% by weight of the hydrophobically modified particles, more preferably from 0.1 to 15%, most preferably from 0.3 to 10%, based on the total weight of the antiperspirant composition and including all ranges subsumed therein.

### Amino Functionalized Silicone

The antiperspirant composition of this invention comprises an amino functionalized silicone. By "amino functionalized silicone" is meant a silicone containing at least one primary, secondary or tertiary amine group, or a quaternary ammonium group.

The primary, secondary, tertiary and/or quaternary amine groups may either form part of the main polymer chain or more preferably be carried by a side or pendant group carried by the polymeric backbone. Such polymers are described, for example, in US 4 185 087.

In a preferred embodiment, the amino functionalized silicone has the general formula: 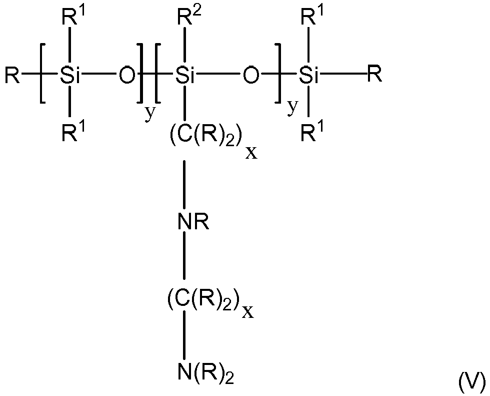 where:
each R is independently H, OH, OCH₃ or C₁₋₄alkyl;
each R¹ is independently OR, or a C₁₋₄alkyl;
each R² is independently OR, or a C₁₋₄ alkyl; and
each x is independently an integer from 1 to 4 and each y is greater than zero and independently an integer to yield a polymer having a molecular weight from 500 to 1 million, and preferably, from 750 to 25,000, and most preferably from 1,000 to 15,000.

Other amino functionalized silicone suitable to use includes silicone cationic polymers represented by the formula:

R²ₐZ₃₋ₐ-Si(OSiZ₂)ₙ-(OSiZ_{b}R²_{2-b})ₘ-O-SiZ₃₋ₐ-R²ₐ (VI)

where:
Z is hydrogen, phenyl, OH or a C₁-C₁₀ alkyl group;
each a is independently an integer from 0 to 3;
b is an integer from 0 to 1; and
m and n are integers whereby the sum of n+m ranges from 1 to 3,500, and preferably from 10 to 160;
each R² is independently a monovalent radical of formula -C_{q}H_{2q}L whre each q is independently a number from 2 to 10 and L is an amine or a quatemized amine represented by one of the following groups:
   - NR³-CH₂-CH₂-N(R³)₂
   - N(R³)₂
   - N^{⊕}(R³)₃A⁻
   - N(R³)-CH₂-CH₂-N⁺R³(H)₂A⁻,
where:
each R³ is independently hydrogen, phenyl, benzyl or a C₁ to C₁₂ alkyl; and
each A⁻ is independently fluoride, chloride, bromide or iodide anion.

Still other amino functionalized silicone suitable for use includes those having the formula: 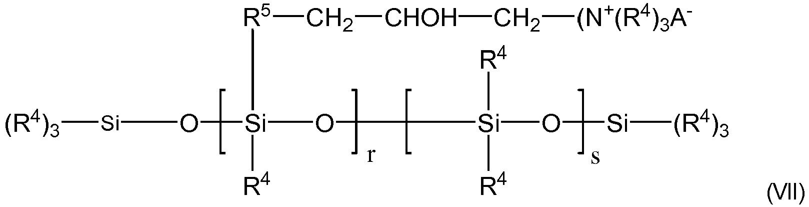 where:
each R⁴ is independently a C₁ to C₂₀ alkyl or C₂ to C₂₀ alkenyl;
R⁵ is a divalent C₁-C₁₈group;
A⁻ is as previously defined;
r is an integer from 2 to 20; and
s is an integer from 15 to 75.

Preferably, the amino functionalized silicone has the INCI designation amodimethicone.

More preferably, the amodimethicone is represented by formula (V) and made commercially available, for example, as DC 8500 by Dow Coming. It is also within the scope of this invention for the amino functionalized silicone to comprise trimethylsilylamodimethicone represented by formula (VI).

Typically, the antiperspirant composition of the present invention comprises amino functionalized silicone in an amount from 0.01 to 20%, more preferably from 0.03 to 15%, more preferably still from 0.05 to 10%, most preferably from 0.1 to 5% by total weight of the antiperspirant composition and including all ranges subsumed therein.

Preferably the weight ratio of hydrophobically modified particle to amino functionalized silicone is from 10:1 to 1:3, more preferably from 5:1 to 1:1.

### Antiperspirant Active

The antiperspirant composition also comprises an antiperspirant active. The antiperspirant actives used herein is aluminum sesquichlorohydrate.

Preferably, the antiperspirant active comprises water soluble calcium salt, such as calcium chloride. It is particularly preferred that the antiperspirant active comprises water soluble calcium salt. Further preferably the antiperspirant active comprises an amino acid, such as glycine. It is especially preferred that the antiperspirant active is heated with the water soluble calcium salt and preferably amino acid prior to or during formulation of the antiperspirant composition. In this way, the antiperspirant active may become 'activated', that is to say, have higher antiperspirant efficacy.

The benefit of the invention is that the combination of the antiperspirant active together with the hydrophobically modified particles and amino functionallised silicone interact synergistically to give enhanced antiperspirant efficacy while ensuring good sensory. This is particularly true with antiperspirant active comprising water soluble calcium salt, especially when such actives also comprise an amino acid, such as glycine.

By enhanced anti-perspirant efficacy above is meant that the efficacy compared to conventional actives can be obtained at relatively low concentrations. This is especially important, as one of the drawbacks of such salts is that they have a tendency to irritate the skin and any active that provides the desired efficacy at lower concentrations would obviously be highly preferred by the consumer as one could get the desired positive benefits without the negatives generally associated with such products.

When calcium-containing/activated antiperspirant actives are employed, it is preferred that the molar ratio of calcium to aluminium is at least 1:20, more preferably at least 1:15 and most preferably at least 1:10. In combination with each of these preferences, when an amino acid such as glycine is also included, it is preferred that the molar ratio of amino acid to aluminium is at least 1:20 and more preferably at least 1:10.

When calcium-containing/activated antiperspirant actives are employed, particularly at any of the preferred molar ratios of calcium to aluminium referred to in the above paragraph and especially at any of both the preferred molar ratios of calcium to aluminium and the preferred molar ratios of amino acid to aluminium referred to in the above paragraph, it is preferred that the antiperspirant active comprises an aluminium sesquichlorohydrate salt of formula Al₂OH_{4.4}Cl_{1.6} to Al₂OH_{4.9}Cl_{1.1} and more preferably of formula Al₂OH_{4.7}Cl_{1.3} to Al₂OH_{4.9}Cl_{1.1}. Such actives are particularly suited to use in the compositions of the present invention.

Typically, the composition comprises antiperspirant active in an amount from 0.1 to 40%, more preferably from 1 to 30%, most preferably from 5 to 20% by total weight of the antiperspirant composition and including all ranges subsumed therein.

Typically, the antiperspirant active is dissolved in the water also present in the composition giving an aqueous solution of the antiperspirant active.

### Other Ingredients

The antiperspirant composition may comprise non-amino functionalized silicone oils as the oil continuous phase. The silicone oils may be volatile or non-volatile. Particularly preferred volatile oils are linear siloxanes containing from 3 to 9 silicon atoms, and cyclic siloxanes having from 4 to 6 silicon atoms such as cyclopentasiloxane. Examples of commercially available volatile silicone oils include oils having grade designations 344, 345, 244, 245 and 246 from Dow corning Corporation.

Suitable non-valatile silicone oils are polyalkylsiloxanes, in particular polydimethylsiloxanes which have the CTFA designation dimethicone that are commercially available under the name Dow Corning 556 and Dow Corning 200 series.

Typically, the antiperspirant composition comprises the non-amino functionalized silicone oil in an amount ranging from 0.05 to 50%, more preferably from 1 to 40%, most preferably from 5 to 30% by total weight of the antiperspirant composition and including all ranges subsumed therein.

The antiperspirant composition may also comprise natural oils that comprise a glyceride of an unsaturated carboxylic acid containing 1, 2, or 3 olefinic bonds. Preferably the unsaturated carboxylic acid containing 1, 2, or 3 olefinic bonds that is part of the glyceride contains from 14 to 22 carbon atoms, more preferably the glyceride is a glyceride of an unsaturated carboxylic acid containing 16-20 carbon atoms, most preferably the glyceride is a glyceride of an unsaturated carboxylic acid containing 18 carbon atoms. Preferably the natural oils comprise triglycerides. Sunflower seed (Helianthus Anuus) oil is the most preferred.

The amount of the natural oils is preferably in the range from 0.1 to 20%, more preferably from 0.5 to 15%, most preferably from 1 to 10% by total weight of the antiperspirant composition and including all ranges subsumed therein.

Humectants may also be included in the antiperspirant composition. Preferably, the humectants are glycerine, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin is particularly preferred. Typically the humectants is employed in an amount from 0.01 to 20%, preferably from 0.1 to 10%, more preferably from 0.5 to 5% by total weight of the antiperspirant composition and including all ranges subsumed therein.

The antiperspirant composition may further comprise fragrance. Suitable fragrance includes socalled deoperfumes as described in EP 545,556 which is hereby incorporated by reference in its entirety. Such fragrance is generally employed from 0.01 to 4%, more preferably from 0.1 to 3%, most preferably from 0.25 to 2% by total weight of the antiperspirant composition.

A deodorant active may also be included in the composition if desired. It is an active other than the antiperspirant salt. Suitable deodorant actives are polyhexamethylene biguanide salts, triclosan, tricloban, chlorhexidine, quaternary ammonium compounds like cetyltrimethylammonium salts, farnesol or mixtures thereof. Such actives are typically incorporated in an amount ranging from 0.01 % to 5%, more preferably from 0.1 to 1% by total weight of the antiperspirant composition.

The viscosity of the antiperspirant composition is typically at least 8000 cP (centipoise=mPa•S) at 25°C, more preferably at least 8500 cP, most preferably from 8500 cP to 15000 cP. The viscosity can be measured by DV-I Viscometer (Brookfield Ltd).

### Method for Manufacturing Antiperspirant Compositions

This invention is also directed to a method for manufacturing an antiperspirant composition. Preferably, the method comprises the steps of:
a) preparing an oil phase comprising hydrophobically modified particles selected from the group consisting of silica, metal oxide and mixtures thereof, and amino functionalized silicone;
b) preparing an aqueous phase comprising antiperspirant active;
c) mixing the two phases to form the antiperspirant composition.

The oil phase, as used herein, refers to a phase comprising water in an amount of less than 1.5%, preferably less than 1.0%, and more preferably less than 0.75% and more preferably still less than 0.5%, and even more preferably less than 0.1% and most preferably from 0.0 to 0.01% by total weight of the oil phase.

The aqueous phase, as used herein, refers to a phase comprising water in an amount of greater than 30%, preferably greater than 35%, and more preferably greater than 40%, and more preferably still greater than 45%, and most preferably from 47% to 70% by total weight of the aqueous phase.

The invention is also concerned with the antiperspirant composition obtainable and/or obtained by the method for manufacturing the antiperspirant composition.

The invention is further concerned with a method of using the antiperspirant composition to reduce perspiration.

The following examples are provided to facilitate an understanding of the present invention. The examples are not provided to limit the scope of the claims.

### Comparative example 1

This example demonstrates the stability of compositions. All ingredients are expressed by weight percent of the total formulation.

Stability, as used herein, refers to the composition maintaining its appearance, odor and macroscopic structure without phase separation.

For stability measurement, the samples were stored in 100mL sealed plastic bottle and put into oven at 50°C. The appearance of samples were observed and recorded after 3 weeks.

For viscosity measurement, the samples were stored in 100mL sealed plastic bottle for at least 30 minutes at 25°C. Their viscosities were measured by DV-I Viscometer (Brookfield Ltd) at a speed of 10 rpm after 10 seconds.

**Table 1**

| Phase | Trade Name | INCI Name | Samples | | | | | |
|---|---|---|---|---|---|---|---|---|
| | | | 1 | 2 | 3 | 4 | 5 | 6 |
| A | Aerosil R805 | Silica | 0.67 | 0.86 | 2.18 | 2.47 | 1.33 | 1.52 |
| | Dow Corning 8500 | Bis(C13-15 Alkoxy) PG Amodimethico ne | 0.52 | 0.51 | 1.02 | 1.00 | 0.68 | 0.67 |
| | PMX-0245 | Cyclopentasilo xane | 13.06 | 12.88 | 25.30 | 25.03 | 16.99 | 16.8 1 |
| B | Chlorhydrol 50% sol | Aluminum Chlorohydrate (50% solution) | 30.00 | 30.00 | 30.00 | 30.00 | 30.00 | 30.0 0 |
| | Pricerine 9091 | Glycerin | 4.00 | 4.00 | 4.00 | 4.00 | 4.00 | 4.00 |
| | Water | Aqua | 46.75 | 46.75 | 32.50 | 32.50 | 42.00 | 42.0 0 |
| C | Akosun | Helianthus Anuus Seed Oil, Citric acid | 4.00 | 4.00 | 4.00 | 4.00 | 4.00 | 4.00 |
| | White Willow G1 | Fragrance | 1.00 | 1.00 | 1.00 | 1.00 | 1.00 | 1.00 |
| Total | | | 100 | 100 | 100 | 100 | 100 | 100 |
| Viscosity/cP | | | 10420 | 12620 | 8920 | 12320 | 11184 | 1346 0 |
| Stability | | | stable | stable | stable | stable | stable | stable |

### Comparative example 2

This example demonstrates the antiperspirant efficacy of compositions. The composition indicated in Table 2 was prepared for evaluation.

**Table 2**

| Trade Name | INCI Name | Amount (%) |
|---|---|---|
| Aerosil R805 | Silica | 1.00 |
| Dow Corning 8500 | Bis(C13-15 Alkoxy) PG Amodimethicone | 0.90 |
| PMX-0245 | Cyclopentasiloxane | 10.00 |
| PMX-200, 5cst | Dimethicone | 8.10 |
| Chlorhydrol 50% sol | Aluminum Chlorohydrate (50% solution) | 30.00 |
| Pricerine 9091 | Glycerin | 4.00 |
| Water | Aqua | 41.00 |
| Akosun | Helianthus Anuus Seed Oil, Citric acid | 4.00 |
| White Willow G1 | Fragrance | 1.00 |
| Total | | 100 |

### Hot Room Test

A conventional roll-ball applicator was used for the applications. Trained operators applied the test composition to a first axilla of panelists once a day for three consecutive days. A control composition was applied to the panelists' second axilla. The sweat measurements were taken 24 hours after the last application. The panelists were required to sit in a hot room (temperature 40°C; relative humidity 40%) for up to one hour and twenty minutes. During the sittings, sweat was collected on absorbent cotton pads held in each axilla and the amount produced was recorded and the result is summarised in Table 3. "Sweat Weight Reduction"(SWR) achieved by the test composition is compared to the control composition (simple ethanolic deodorant without antiperspirant active).

**Table 3**

| Time | SWR (%) |
|---|---|
| 4 days | 27 |

Table 3 shows that the test composition achieves significant SWR.

### Comparative example 3

This example demonstrates mildness of compositions.

### Irritation Test

Panelists were recruited for the study. The duration of the study was 12 days in total including two phases. Phase 1 is the Provocation period (days 1-5) with daily shaving the volar forearms to induce mild irritation and erythema. Phase 2 is the Recovery period (days 6-12) in which the panelists were required to apply the test compositions twice daily during a six-day recovery phase. Measurement for hydration using the Corneometer was carried out on days 6, 7, 8, 9 and 12.

Comparative sample A is a mild antiperspirant roll-on formulation from the prior art, known to have good skin care credentials, which comprises aqua, aluminium chlorohydrate, steareth-2, parfum, steareth-20, helianthus annuus seed oil, glycerine and tocopheryl acetate. Comparative sample B is a marketed antiperspirant roll-on formulation, claimed to have good skin care credentials, which comprises aqua, aluminium chlorohydrate, PPG-15 stearyl ether, steareth-2, steareth-21, parfum, aluminium sesquichlorohydrate, magnesium aluminium silicate, persea gratissima oil and trisodium EDTA. Sample C is the composition as indicated in Table 2.

Tests were conducted as: 1) within each product, to compare day 6 response levels to days 7, 8, 9 and 12; 2) for each time point post-baseline (days 7, 8, 9 and 12), to analyse differences between each of the products response.

Day 6 data was treated as a baseline covariate, and confidence intervals around the estimates are presented at the 90% confidence level.

The relative hydration results measured by corneometer are shown in Table 4. The column "B-A" is the results for corneometer level of B minus those of A and the column "A-C" is the results for corneometer level of A minus those of C.

**Table 4**

| Time | Comparison | |
|---|---|---|
| | B-A | A-C |
| 7 days | -1.9103 | -2.5045 |
| 8 days | -1.391 | -3.5421 |
| 9 days | -2.3009 | -3.0845 |
| 12 days | -3.0336 | -1.2422 |

Table 4 shows the relative hydration levels between the samples, the higher the hydration the lower the irritation. It can be seen from the table that Sample B which is a marketed antiperspirant roll-on formulation shows the lowest level of hydration. Sample C according to the present invention shows significantly better hydration than Sample A.

### Example-4:

This example demonstrates the antiperspirant efficacy of a composition as per the invention. The composition indicated in Table 5 was prepared for evaluation.

**Table 5**

| Trade Name | INCI Name | Amount (%) |
|---|---|---|
| HDK H30 | Silica Dimethyl Silylate | 0.60 |
| Dow Corning 8500 | Bis(C13-15 Alkoxy) PG Amodimethicone | 0.577 |
| PMX-0245 | Cyclopentasiloxane | 14.81 |
| Reach 301 (40% anhydrous ASCH) | Aluminum Sesquihlorohydrate | 30.00 |
| Pricerine 9091 | Glycerin | 4.00 |
| Water | Aqua | 41.80 |
| Akosun | Helianthus Anuus Seed Oil, Citric acid | 4.00 |
| White Willow G1 | Fragrance | 1.00 |
| Calcium Chloride Dihydrate | Calcium chloride | 1.22 |
| Glycine | Glycine | 2.00 |
| Total | | 100 |

### Hot Room Test

SWR was determined using the same procedure as used for Example - 2 and the result is summarised in Table - 6.

**Table 6**

| Time | SWR (%) |
|---|---|
| 4 days | 64 |

Table 3 shows that the test composition achieves significantly better SWR as compared to a composition without anti-perspirant active and also significantly superior SWR as compared to a composition comprising aluminium chlorohydrate (Example - 2).

### Example -5:

Sensory evaluation of a composition as per the invention as compared to a conventional composition comprising an antiperspirant active (ACH).Composition as shown in Table -5 above was used by 32 female subjects in comparison to a conventional antiperspirant composition comprising aluminium chlorohydrate (ACH). They rated the two compositions on various sensorial properties and the data is summarized in Table- 7 below:

| Attribute | Comparitive sensorial benefit of composition of Table -5 v/s control |
|---|---|
| Wetness | = |
| Silkiness | ++ |
| Smoothness | ++ |
| Absorbancy | = |
| Visibility | = |
| Glidability | ++ |
| Stickiness (intial) | ++ |
| Stickiness (overtime) | ++ |
| Comfort | ++ |
| Oiliness | = |
| Drying time | + |

| | |
|---|---|
| ++ means significantly better + means directionally better = means parity - means directionally worse - means significantly worse | |

The data in Table 7 above indicates that composition as per the invention is on parity compared to a conventional composition on most attributes and significantly better on some attributes.

## Claims

1. An antiperspirant composition comprising:
a) hydrophobically modified particles selected from the group consisting of silica, metal oxide and mixtures thereof;
b) amino functionalized silicone;
c) water; and
d) an antiperspirant active,
wherein the antiperspirant active is aluminum sesquichlorohydrate.

2. The antiperspirant composition according to claim 1, wherein the composition is substantially free of emulsifiers.

3. The antiperspirant composition according to claim 1 or claim 2, wherein the hydrophobically modified particle is hydrophobically modified silica.

4. The antiperspirant composition according to any of the preceding claims, wherein the hydrophobically modified silica comprises: 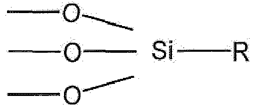 wherein R is a C₄ to C₁₈ alkyl group, preferably R is C₈H₁₇ alkyl group.

5. The antiperspirant composition according to any of the preceding claims, wherein the amino functionalized silicone comprises amodimethicone.

6. The antiperspirant composition according to any of the preceding claims, wherein the composition comprises amino functionalized silicone in an amount from 0.01 to 20%, preferably from 0.05 to 1 0% by total weight of the composition.

7. The antiperspirant composition according to any of the preceding claims, wherein the weight ratio of hydrophobically modified particle to amino functionalized silicone is from 10:1 to 1:3, preferably from 5:1 to 1:1.

8. The antiperspirant composition according to any of preceding claims, wherein the antiperspirant active comprises a water soluble calcium salt.

9. The antiperspirant composition according to claim 8 additionally comprising an amino acid preferably glycine.

10. The antiperspirant composition according to any of the preceding claims, wherein the composition comprises antiperspirant active in an amount from 0.1 to 40%, preferably from 5 to 20% by total weight of the composition.

11. The antiperspirant composition according to any of the preceding claims, wherein the composition further comprises non-amino functionalized silicone, preferably cyclopentasiloxane.

12. The antiperspirant composition according to any of the preceding claims, wherein the composition further comprises natural oil, preferably sunflower seed oil.

13. The antiperspirant composition according to any of the preceding claims, wherein the composition further comprises fragrance.

14. An antiperspirant product comprising the antiperspirant composition as claimed in any one of claims 1 to 13, wherein the antiperspirant composition is contained in a roll-on dispenser.

15. A process for manufacturing the antiperspirant composition as claimed in any one of claims 1 to 13 wherein the process comprises the steps of:
a) preparing an oil phase comprising hydrophobically modified particles selected from the group consisting of silica, metal oxide and mixtures thereof, and amino functionalized silicone;
b) preparing an aqueous phase comprising antiperspirant active;
c) mixing the two phases to form the antiperspirant composition.

## Patentansprüche

1. Schweißhemmende Zusammensetzung, umfassend:
a) hydrophob modifizierte Partikel, ausgewählt aus der aus Siliziumdioxid, Metalloxid und Mischungen davon bestehenden Gruppe,
b) amino-funktionalisiertes Silikon,
c) Wasser und
d) einen schweißhemmenden Wirkstoff,
wobei der schweißhemmende Wirkstoff Aluminiumsesquichlorhydrat ist.

2. Schweißhemmende Zusammensetzung nach Anspruch 1, wobei die Zusammensetzung im Wesentlichen frei von Emulgatoren ist.

3. Schweißhemmende Zusammensetzung nach Anspruch 1 oder Anspruch 2, wobei die hydrophob modifizierte Partikel hydrophob modifiziertes Siliziumdioxid ist.

4. Schweißhemmende Zusammensetzung nach einem der vorhergehenden Ansprüche, wobei das hydrophob modifizierte Siliziumdioxid umfasst: 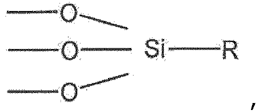 wobei R eine C₄- bis C₁₈-Alkyl-Gruppe ist, vorzugsweise R eine C₈H₁₇-Alkyl-Gruppe ist.

5. Schweißhemmende Zusammensetzung nach einem der vorhergehenden Ansprüche, wobei das amino-funktionalisierte Silikon Amodimethicon umfasst.

6. Schweißhemmende Zusammensetzung nach einem der vorhergehenden Ansprüche, wobei die Zusammensetzung amino-funktionalisiertes Silikon in einer Menge von 0,01 bis 20%, vorzugsweise von 0,05 bis 10%, bezogen auf das gesamte Gewicht der Zusammensetzung, umfasst.

7. Schweißhemmende Zusammensetzung nach einem der vorhergehenden Ansprüche, wobei das Gewichtsverhältnis von hydrophob-modifizierter Partikel zu amino-funktionalisiertem Silikon von 10:1 bis 1:3, vorzugsweise von 5:1 bis 1:1, beträgt.

8. Schweißhemmende Zusammensetzung nach einem der vorhergehenden Ansprüche, wobei der schweißhemmende Wirkstoff ein wasserlösliches Calciumsalz umfasst.

9. Schweißhemmende Zusammensetzung nach Anspruch 8, die zusätzlich eine Aminosäure, vorzugsweise Glycin, umfasst.

10. Schweißhemmende Zusammensetzung nach einem der vorhergehenden Ansprüche, wobei die Zusammensetzung schweißhemmenden Wirkstoff in einer Menge von 0,1 bis 40%, vorzugsweise von 5 bis 20%, bezogen auf das gesamte Gewicht der Zusammensetzung, umfasst.

11. Schweißhemmende Zusammensetzung nach einem der vorhergehenden Ansprüche, wobei die Zusammensetzung ferner nicht-amino-funktionalisiertes Silikon, vorzugsweise Cyclopentasiloxan, umfasst.

12. Schweißhemmende Zusammensetzung nach einem der vorhergehenden Ansprüche, wobei die Zusammensetzung ferner natürliches Öl, vorzugsweise Sonnenblumenkernöl, umfasst.

13. Schweißhemmende Zusammensetzung nach einem der vorhergehenden Ansprüche, wobei die Zusammensetzung ferner Duftstoff umfasst.

14. Schweißhemmendes Produkt, umfassend die schweißhemmende Zusammensetzung, wie in irgendeinem der Ansprüche 1 bis 13 beansprucht, wobei die schweißhemmende Zusammensetzung in einem Roll-on-Spender enthalten ist.

15. Verfahren zur Herstellung der schweißhemmenden Zusammensetzung, wie in irgendeinem der Ansprüche 1 bis 13 beansprucht, wobei das Verfahren die Schritte umfasst:
a) Herstellen einer Öl-Phase, umfassend hydrophob modifizierte Partikel, ausgewählt aus der Gruppe, die aus Siliziumdioxid, Metalloxid und Mischungen davon besteht, und amino-funktionalisiertes Silikon,
b) Herstellen einer wässrigen Phase, umfassend schweißhemmenden Wirkstoff,
c) Mischen der zwei Phasen, um die schweißhemmende Zusammensetzung zu bilden.

## Revendications

1. Composition d'antiperspirant comprenant :
a) des particules hydrophobiquement modifiées choisies dans le groupe constitué de silice, d'oxyde de métal et de mélanges de ceux-ci ;
b) du silicone amino fonctionnalisé ;
c) de l'eau ; et
d) un actif d'antiperspirant,
dans laquelle l'actif d'antiperspirant est le sesquichlorohydrate d'aluminium.

2. Composition d'antiperspirant selon la revendication 1, dans laquelle la composition est pratiquement exempte d'émulsionnants.

3. Composition d'antiperspirant selon la revendication 1 ou la revendication 2, dans laquelle la particule hydrophobiquement modifiée est de la silice hydrophobiquement modifiée.

4. Composition d'antiperspirant selon l'une quelconque des revendications précédentes, dans laquelle la silice hydrophobiquement modifiée comprend : 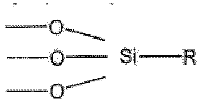 dans laquelle R est un groupe alkyle en C₄ à C₁₈, R est de préférence un groupe alkyle C₁₈H₁₇.

5. Composition d'antiperspirant selon l'une quelconque des revendications précédentes, dans laquelle le silicone amino fonctionnalisé comprend de l'amodiméthicone.

6. Composition d'antiperspirant selon l'une quelconque des revendications précédentes, dans laquelle la composition comprend du silicone amino fonctionnalisé dans une quantité de 0,01 à 20 %, de préférence de 0,05 à 10 % en masse totale de la composition.

7. Composition d'antiperspirant selon l'une quelconque des revendications précédentes, dans laquelle le rapport massique de particules hydrophobiquement modifiées à silicone amino fonctionnalisé est de 10:1 à 1:3, de préférence de 5:1 à 1:1.

8. Composition d'antiperspirant selon l'une quelconque des revendications précédentes, dans laquelle l'actif d'antiperspirant comprend un sel de calcium soluble dans l'eau.

9. Composition d'antiperspirant selon la revendication 8, comprenant de plus un acide aminé de préférence la glycine.

10. Composition d'antiperspirant selon l'une quelconque des revendications précédentes, dans laquelle la composition comprend un actif d'antiperspirant dans une quantité de 0,1 à 40 %, de préférence de 5 à 20 % en masse totale de la composition.

11. Composition d'antiperspirant selon l'une quelconque des revendications précédentes, dans laquelle la composition comprend de plus du silicone non-amino fonctionnalisé, de préférence du cyclopentasiloxane.

12. Composition d'antiperspirant selon l'une quelconque des revendications précédentes, dans laquelle la composition comprend de plus une huile naturelle, de préférence de l'huile de graine de tournesol.

13. Composition d'antiperspirant selon l'une quelconque des revendications précédentes, dans laquelle la composition comprend de plus du parfum.

14. Produit d'antiperspirant comprenant la composition d'antiperspirant selon l'une quelconque des revendications 1 à 13, dans laquelle la composition d'antiperspirant est contenue dans un distributeur à bille.

15. Procédé de fabrication de la composition d'antiperspirant selon l'une quelconque des revendications 1 à 13, dans lequel le procédé comprend les étapes de :
a) préparation d'une phase d'huile comprenant des particules hydrophobiquement modifiées choisies dans le groupe constitué de silice, d'oxyde de métal et de mélanges de ceux-ci, et du silicone amino fonctionnalisé ;
b) préparation d'une phase aqueuse comprenant un actif d'antiperspirant ;
c) mélange des deux phases pour former la composition d'antiperspirant.
